# EUROPEAN PATENT SPECIFICATION

(11) **EP 0 523 743 B1**
(45) Date of publication and mention of the grant of the patent: **17.04.1996**
(21) Application number: 92112377.4
(22) Date of filing: 20.07.1992
(51) Int. Cl.: A61L 17/00, D02G 3/36, A61B 17/06

(54) **Sterilized bicomponent fiber braids**
Sterilisierte Zweikomponenten-Fasernlitze
Toron stérilisé de fibres à deux composants

(30) Priority: 18.07.1991 US 732371
(43) Date of publication of application: 20.01.1993
(73) Proprietor: ETHICON INC., Somerville New Jersey 08876 (US)
(72) Inventor: Steckel, Mark, Flemington, NJ 08822 (US); Price, Lester F., Monmouth Jct., NJ 08852 (US)
(74) Representative: Strehl Schübel-Hopf Groening & Partner

(56) References cited:
- EP-A- 0 448 840
- FR-A- 2 096 931

## Description

### BACKGROUND OF THE INVENTION

This invention relates to braided multifilaments, and especially to sterilized, braided multifilaments suitably adapted for use as surgical sutures or ligatures.

Braided multifilaments often offer enhanced pliability, knot security and tensile strength when compared to their monofilament counterparts. The enhanced pliability of a braided multifilament is a direct consequence of the lower resistance to bending of a bundle of very fine filaments relative to one large diameter monofilament. However, for this enhancement to be realized, the individual multifilaments must be able to bend unencumbered or unrestricted by their neighboring filaments. Any mechanism which reduces this individual fiber mobility, such as simple fiber-fiber friction, a coating which penetrates into the braid interstices, or a melted polymer matrix which adheres fibers together, will adversely affect braid pliability. In the extreme case where the multifilaments are entirely bonded together, the pliability or bending resistance is essentially equivalent to a monofilament.

Unfortunately, the prior art abounds with attempts to improve specific properties of multifilament braids at the expense of restricting the movement of adjacent filaments which make up the braid. For example, multifilament sutures almost universally possess a surface coating to improve handling properties.
U.S. Patent 3,942,532 discloses a polyester coating for multifilament sutures. The preferred polyester coating is polybutylate, which is the condensation product of 1,4-butanediol and adipic acid. U.S. Patent 4,624,256 discloses a suture coating copolymer of at least 90 percent ε-caprolactone and a biodegradable monomer, and optionally a lubricating agent. Examples of monomers for biodegradable polymers disclosed include glycolic acid and glycolide, as well as other well known monomers typically used to prepare bioabsorbable coatings for multifilament sutures.

An alternative to the use of the commonly accepted coating compositions for multifilament sutures to improve handling properties is disclosed in U.S. Patent 3,527,650. This patent discloses a coating composition of polytetrafluoroethylene (PTFE) particles in an acrylic latex. Although the PTFE particles act as an excellent lubricant to decrease the surface roughness of multifilament sutures, the particles have a tendency to flake off during use. Also, this particular coating is a thermoset which requires a curing step for proper application.

More recently, a dramatic attempt has been made to create a monofilament-like surface for a multifilament suture. U.S. Patent 4,470,941 discloses the preparation of "composite" sutures derived from different synthetic polymers. The composite suture is composed of a core of low melting fibers around which are braided high melting fibers. Because of the lack of cohesiveness of the dissimilar fibers, the low melting fibers in the core are melted and redistributed throughout the matrix of the braided, high melting fibers. Although these composite sutures represent an attempt to combine the best properties of different synthetic fibers, it unfortunately fails in this respect because the redistributed, low melting fibers form a matrix which significantly increases the stiffness of the composite suture by reducing fiber mobility.

Another attempt to enhance the properties of multifilament sutures can be found in WO 86/00020. This application discloses coating an elongated core of a synthetic polymer having a knot tenacity of at least 7 grams 0,111 tex (denier) with a film-forming surgical material. The film-forming surgical material can be absorbable or nonabsorbable, and can be coated on the elongated core by solution coating, melt coating or extrusion coating. Such coated multifilament sutures suffer from the same deficiencies which plague conventionally coated multifilament sutures.

All of the attempts described in the prior art to improve braid properties have overlooked the importance of fiber-fiber friction and its impact on fiber mobility and braid pliability. The properties of concern here include the fiber-fiber frictional coefficients (which frequently relate to the polymer's surface energy), the fiber cross-sectional shape and diameter, and the braid structure which influences the transverse forces across the braid. If fibers composed of highly lubricous polymers are used in the traditional manner, then a highly pliable braid can be prepared. However, in most cases, these braids will be relatively weak and unusable. Hence, a tradeoff between braid strength and pliability exists in the design of conventional braided multifilaments.

In view of the deficiencies of the prior art, it would be desirable to prepare multifilament sutures exhibiting improved pliability and handling properties. More specifically, it would be most desirable to prepare braided multifilaments composed of fibers having at least two dissimilar fiber-forming materials in which the fiber-forming materials contribute significantly to enhanced pliability for the braided multifilament without appreciably sacrificing its physical properties.

### SUMMARY OF THE INVENTION

The invention is a bicomponent fiber braid comprising sterilized, braided, continuous and discrete yarns. Each yarn is composed of a plurality of filaments, and at least one filament from one or more yarns is a bicomponent fiber. The bicomponent fiber possesses a sheath of a first fiber-forming material enclosing along the length of the fiber a core of a second fiber-forming material.

Suprisingly, the bicomponent fiber braids may exhibit a combination of outstanding properties attributable to the specific properties of the dissimilar fiber-forming materials which make up the braided yarns. The dissimilar fiber-forming materials do not require melt bonding or any other special processing techniques to prepare the biocomponent fibers which make up the braid of this invention. Instead, the integrity of the braid and therefore its properties is due entirely to the mechanical interlocking or weaving of the individual yarns. In fact, it is possible to tailor the physical and biological properties of the braid by varying the type and proportion of each of the dissimilar fiber-forming materials used, as well as by adjusting the specific configuration of the braid. For example, in preferred embodiments, the bicomponent fiber braid will exhibit improved pliability relative to that of braids prepared from conventional monolithic fiber braids, without significantly sacrificing physical strength or knot security.

The sterilized, bicomponent fiber braids of this invention are useful as surgical sutures or ligatures, as well as for the preparation of any other medical device which would benefit from its outstanding physical or biological properties.

### BRIEF DESCRIPTION OF THE DRAWINGS

Figure 1 is a photomicrograph illustrating the bicomponent fiber braid prepared in Example 1 in perspective at a magnification of 50X.

Figure 2 is a photomicrograph illustrating the bicomponent fiber braid of Figure 1 in cross section at a magnification of 100X, and further illustrating at a higher magnification of 1000X a plurality of the individual bicomponent fibers of the braid.

Figure 3 is a photomicrograph illustrating the individual bicomponent fibers of Figure 2 in cross section at a magnification of 1500X.

### DETAILED DESCRIPTION OF THE INVENTION

For purposes of describing this invention, a "bicomponent fiber" braid is a configuration of sterilized, braided yarns in which one or more yarns is composed of at least one bicomponent fiber. A bicomponent fiber is a continuous fiber having a tubular sheath of a first fiber-forming material enclosing along the length of the fiber a central core of a second, longitudinally extending fiber-forming material.

Each yarn in the braided configuration is composed of a plurality of filaments. Preferably, each of the yarns is composed of a plurality of bicomponent fibers. The bicomponent fibers which can be used to prepare the braided yarns of the invention can be prepared conventionally by coextrusion through a two cavity die as described, for example, in European Patent Application 0 277 707 and the Encyclopedia of Textiles, Fibers, and Nonwoven Fabrics, John Wiley & Sons, New York (1984), pp. 152-172 and pp. 347-381.

The sterilized, braided yarns which make up the bicomponent fiber braids are continuous and discrete, so therefore each yarn extends substantially along the entire length of the braid and maintains its individual integrity during braid preparation, processing and use.

The bicomponent fiber braids of this invention can be conventionally braided in a tubular sheath around a core of longitudinally extending yarns, although such a core may be excluded, if desired. Braided sheath sutures with central cores are shown in U.S. Patent Nos. 3,187,752; 4,043,344; and 4,047,533, for example. A core may be advantageous because it can provide resistance to flattening, as well as increased strength.

The first and second fiber-forming materials which make up the bicomponent fibers of the braided yarns can be any materials capable of being spun into continuous filaments. Advantageously, the fiber-forming materials are nonmetallic.

The preferred fiber-forming materials are synthetic fiber-forming polymers which are capable of being melt spun through a spinneret to prepare continuous filaments. The fiber-forming polymers can be bioabsorbable or nonabsorbable, depending on the particular application desired. Examples of monomers from which bioabsorbable polymers are derived include, but are not limited to, lactides and lactones, e.g. glycolic acid, glycolide, lactide, p-dioxanone, ε-caprolactone and trimethylene carbonate, as well as copolymers and polymer blends derived from these monomers and others. Interestingly, numerous bioabsorbable bicomponent fiber braids exhibiting varying biological properties, such as the breaking strength in vivo and the absorption profile in vivo, can be prepared for specific applications by using bicomponent fibers with different combinations of bioabsorbable polymers. For example, a braid consisting of bicomponent fibers with a sheath of a slow degrading polymer, such as polydioxanone, and a core of a faster degrading polymer, such as a homopolymer of glycolide or a copolymer of glycolide and lactide, can exhibit enhanced strength retention versus conventional absorbable braided sutures.

Preferably, the dissimilar fiber-forming polymers which make up the braided yarns are derived from nonabsorbable polymers. In a preferred embodiment, the first fiber-forming polymer which makes up the fiber sheath exhibits lubricating properties to improve the overall pliability, or compliance, and surface tactile properties of the bicomponent fiber braid. Preferably, continuous filaments prepared from the first fiber-forming polymer exhibit a surface energy less than about 38µN (dyne)/cm, as measured by contact angle of liquids on polymer surfaces, as described by Kissa, E., "Handbook of Fiber Science and Technology," Vol. II, Part B, Marcel Decker, 1984. Such fiber-forming polymers include polypropylene (PP); polyethylene; perfluorinated polymers, e.g. polytetrafluoroethylene (PTFE) and fluorinated ethylene/propylene copolymer (FEP); polyvinylidene fluoride, polyethylene/tetrafluoroethylene copolymers (PETFE), and polychlorofluoroethylene. More preferably, continuous filaments prepared from the first fiber-forming polymer exhibit a surface energy less than about 30µN (dyne)/cm. The preferred first fiber-forming polymers are PP, PETFE, and PTFE, and the most preferred fiber-forming polymer is PP.

In a more preferred embodiment, the bicomponent fibers possess a lubricating sheath in combination with a tenacious core which acts to provide improved strength to the individual bicomponent fibers and the braided yarns.

Preferably, continuous filaments prepared from the second fiber-forming polymer which makes up the fiber core would exhibit a tenacity greater than 3.5 grams per 0.111 tex (denier), more preferably greater than 5.0 grams per 0.111 tex (denier).

The preferred second fiber-forming polymers are polyethylene terephthalate (PET) and polyamide (nylon), and the most preferred polymer is PET.

In the most preferred embodiment, the braided yarns are composed of bicomponent fibers in which each fiber possesses a PP sheath and a PET core. Advantageously, the yarns are constructed in a tubular sheath configuration around a core of longitudinally extending yarns to further improve the overall strength of the bicomponent fiber braid. These longitudinally extending yarns are preferably composed of either homogeneous PET fibers or bicomponent fibers in a PP sheath/PET core configuration. In either embodiment, the volume fraction of each yarn of PP in the braided sheath and core desirably ranges from about 10 to about 60 percent, preferably from about 30 to about 50 percent. A volume fraction of PP below about 10 percent may affect the pliability of the braid and increase processing difficulties, and a volume fraction above about 60 percent may adversely affect the overall strength of the braid. The filament fineness for such a bicomponent fiber braid is preferably between about 0.055 tex (0.5 denier) to about 0.333 tex (3.0 denier) per filament. A more coarse filament may result in a stiffer braid.

The bicomponent fibers prepared for the braids of the invention are bundled together in conventional fashion to prepare the yarns. Preferably, the yarn fineness is between about 1.11 to about 11.1 tex (10 to about 100 denier). The yarns are advantageously stretched to provide molecular orientation, annealed to enhance dimensional stability, and wound on bobbins for eventual braid preparation. The bicomponent fiber braids can then be prepared using conventional braiding technology and equipment commonly used in the textile industry, and in the medical industry for preparing multifilament sutures.

Similar to the preparation of conventional homogeneous braids, the yarns from which the bicomponent fiber braids are prepared are preferably nontextured. The number of picks per 25.4 mm (inch) in the finished braid can be adjusted to balance the tensile strength and the diameter of the braid with braid quality, e.g the tendency for "core popping", which is the tendency of core yarns to break through the braided sheath as the braid is bent, and overall braid smoothness.

After the bicomponent fiber braid is prepared, it is desirably scoured to remove machine oils, and any foreign particles. The scoured braid is preferably stretched at a temperature between the glass transition temperature and melting temperature of the lower melting fiber-forming material. This stretching operation densifies the braid and improves braid smoothness. Afterwards, the braid can be annealed while under restraint to improve dimensional stability, and in the case of absorbable braids, to improve the breaking strength retention in vivo.

If desired, the surface of the bicomponent fiber braid can be coated with a bioabsorbable or nonabsorbable coating to further improve the handleability and knot tiedown performance of the braid. For example, the braid can be immersed in a solution of a desired coating polymer in an organic solvent, and then dried to remove the solvent.

The post-treated bicomponent fiber braid is sterilized so it can be used for a host of medical applications, especially for use as a surgical suture or ligature. The braid can be sterilized using any of the conventional techniques well known in the art. For example, sterilization can be effected by exposing the braid to gamma radiation from a cobalt 60 source. Alternatively, if such a radiation technique undesirably degrades the braid, then the braid can be sterilized by exposure to ethylene oxide.

In the following examples, the tensile properties and knot security are each determined using an Instron Tensile Tester. The tensile properties, i.e. the straight and knot tensile strength, are determined generally according to the procedures described in U.S. Patent 4,838,267. The knot security, which provides an indication as to the number of throws required to secure a knot so that it fails to slip before cleanly breaking, is measured by first tieing a conventional square knot around a mandrel, pulling the knot apart on the Instron Tester to observe whether slipping occurs, and if so, then tieing knots with additional throws until 20 out of 20 knots break cleanly without slipping. The bending rigidity, which is the inverse of pliability, is determined using a Kawabata Pure Bending Tester, as discussed in "The Effects of Structure on the Geometric and Bending Properties of Small Diameter Braids", Drexel University Master Thesis, 1991, by Mr. E. Ritter.

The types of fiber-forming materials used to prepare the bicomponent fiber braid and the yarn geometry can be varied to prepare bicomponent fiber braids within the scope of the claimed invention which exhibit a combination of outstanding physical or biological properties.

### EXAMPLE I

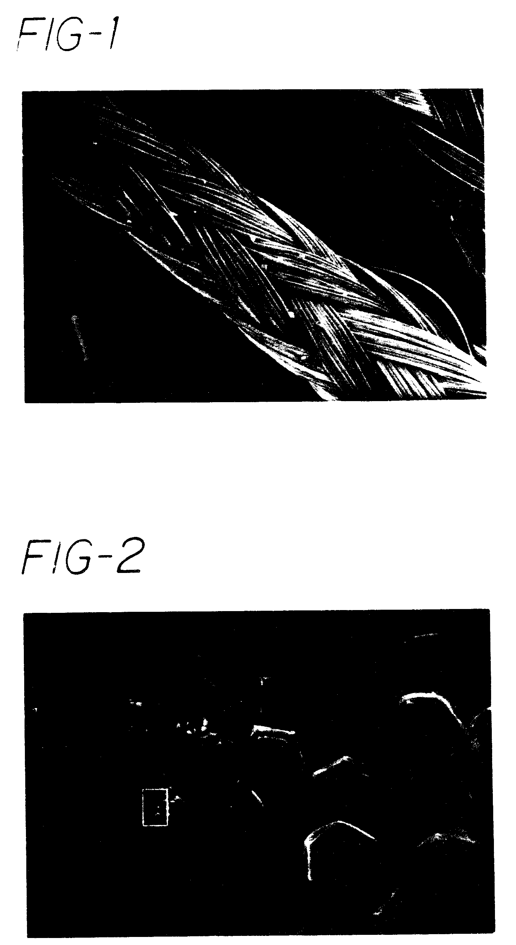

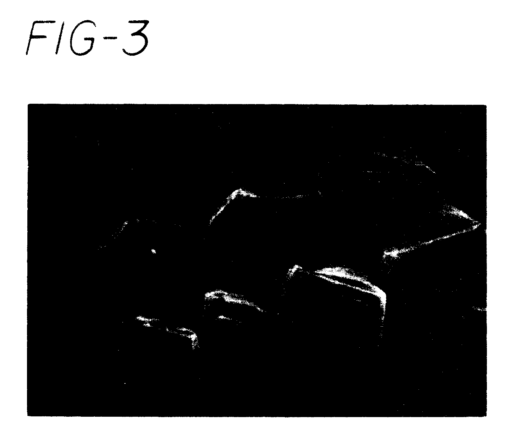

FIBER MATERIALS: A PET/PP (core/sheath) bicomponent fiber is prepared by coextrusion and subsequent orientation. The structure of the coextruded bicomponent fiber is shown in cross-section in Figure 3. The relative volume fractions of the PP and PET are 50/50. The draw ratio during orientation is 3.0 at a godet temperature of 68.33°C (155°F), resulting in a 3.996 tex (36 denier) yarn. The drawn fiber is wound on braider bobbins per conventional methods.
PROCESSING: A 3.996 tex (36 denier) yarn is placed on each bobbin carrier of a 16 carrier N.E. Butt braider, and four 3.996 tex (36 denier) yarns are placed on the core let off device. A 35 pick gear is used resulting in 38 picks per 25.4 mm (inch). No spring tension is applied to the sheath yarns, and the core tension is maintained at 15-20 grams. The braid is aqueous scoured and hot stetched at 5% stretch ratio (51.67°C, 27.43 m/min 〈125°F, 90 fpm〉) over a hot plate assembly. The hot-stretched braid is passed through pliabilizing pins at 27.43 m/min (90 fpm).
PROPERTIES: The resulting braid possesses a diameter of 0.310 mm (0.0122 inches), hence a United States Pharmaecopia (USP) size 2-0 nonabsorbable suture. The braid and the individual bicomponent fibers are shown in perspective and cross-section in Figures 1 and 2, respectively. The pliability of the bicomponent fiber composite braid, as measured by the Kawabata Pure Bending Tester, is compared with the two leading nonabsorbable braided USP size 2-0 sutures, PET and silk, in Table 1. The data represents the engineering bending rigidity of an individual braided suture strand. The bending rigidity is the inverse of the subjective property pliability, so a lower value indicates a more pliable braid.

**TABLE 1**

| PLIABILITY OF BICOMPONENT FIBER BRAID | |
|---|---|
| BRAID TYPE (Size 2-0) | BENDING RIGIDITY (GRAMS X CM²/STRAND) |
| EXAMPLE I PET/PP BICOMPONENT FIBER BRAID | 0.00998 |
| PET BRAIDED SUTURE | 0.0725 |
| SILK BRAIDED SUTURE | 0.113 |

The results indicated in Table 1 demonstrate the dramatically improved pliability of a bicomponent fiber braid relative to the pliability exhibited by conventional braids. In addition, the bicomponent fiber braid of EXAMPLE I demonstrates a tensile strength of 2.631 kg (5.8 lbs) and knot strength of 1.859 kg (4.1 lbs), and a knot security of 4 throws. These values exceed the USP (United States Pharmacopia) requirements for size 2-0 nonabsorbable sutures. These results indicate that the improvement in pliability can be achieved without unacceptable sacrifices in physical properties.

The bicomponent fiber braid of this EXAMPLE I is sterilized by Co⁶⁰ using conventional methods, and subsequently evaluated for subjective handling properties versus the PET and silk braids. The averages of the ratings were PP/PET 1.5, PET 3.3, silk 4.1, where 1 represents the best handling properties and 5 is the worst.

### EXAMPLE II

FIBER MATERIALS: Same as EXAMPLE I, except that a two ply 3,996 tex (36 denier) yarn (2/36) is wound on each braider bobbin.
PROCESSING: The 2/3.996 tex (36 denier) plied yarn is placed on each bobbin carrier of an 8 carrier N.E. Butt braider, and one 2/3.996 tex (36 denier) plied yarn is placed on the core let off device. A 32 pick gear is used resulting in 42 picks per 25.4 mm (inch). No spring tension is applied to the sheath yarns, the core tension is maintained at 15-20 grams. The braid is aqueous scoured and hot stretched at 15% (51.67°C (125°F), 27.43 m/min (90 fpm)) over a hot plate assembly.
PROPERTIES: The resulting braid possesses a diameter of 0.2997 mm (0.0118 inches), hence also a USP size 2-0 non-absorbable suture. The braid demonstrates a bending rigidity of 0.0184 gms.cm /strand, tensile strength of 2.540 kg (5.6 lbs), and a knot strength of 1.769 kg (3.9 lbs).
STERILIZATION: The bicomponent fiber braid can be sterilized using standard techniques described in the specification. After sterilization, the braid still exhibits improved pliability without an appreciable loss of its physical properties.

## Claims

1. A bicomponent fiber braid comprising sterilized, braided, continuous and discrete yarns, each yarn is composed of a plurality of filaments, and at least one filament from one or more yarns is a bicomponent fiber wherein the fiber possesses a sheath of a first fiber-forming material enclosing along the length of the fiber a core of a second fiber-forming material.

2. The bicomponent fiber braid of claim 1 wherein each yarn is composed of a plurality of bicomponent fibers.

3. The bicomponent fiber braid of claim 2 wherein the first and second fiber-forming materials are nonmetallic.

4. The bicomponent fiber braid of claim 3 wherein the first and second fiber-forming materials are synthetic fiber-forming polymers.

5. The bicomponent fiber braid of claim 4 wherein the fiber-forming polymers are nonabsorbable.

6. The bicomponent fiber braid of claim 1 wherein the first fiber-forming polymer is polypropylene (PP), polyethylene/tetraflouroethylene copolymers (PETFE) or polytetrafluoroethylene (PTFE).

7. The bicomponent fiber braid of claim 6 wherein the first fiber-forming polymer is polypropylene (PP).

8. The bicomponent fiber braid of claim 7 wherein a continuous filament prepared from the second fiber-forming polymer exhibits a tenacity greater than 5.0 grams/0.111 tex (denier).

9. The bicomponent fiber braid of claim 8 wherein the second fiber-forming polymer is polyethylene terephthalate (PET) or polyamide (nylon).

10. The bicomponent fiber braid of claim 9 wherein the second fiber-forming material is polyethylene terephthalate (PET).

## Patentansprüche

1. Bikomponentenfasergeflecht, umfassend sterilisierte, geflochtene, kontinuierliche und diskrete Garne, wobei jedes Garn aus einer Vielzahl von Filamenten zusammengesetzt ist und mindestens ein Filament eines oder mehrerer Garne eine Bikomponentenfaser ist, wobei die Faser einen Mantel aus einem ersten faserbildenden Material aufweist, der entlang der Faser einen Kern aus einem zweiten faserbildenden Material einschließt.

2. Bikomponentenfasergeflecht nach Anspruch 1, wobei jedes Garn aus einer Vielzahl von Bikomponentenfasern zusammengesetzt ist.

3. Bikomponentenfasergeflecht nach Anspruch 2, wobei die ersten und zweiten faserbildenden Materialien nichtmetallisch sind.

4. Bikomponentenfasergeflecht nach Anspruch 3, wobei die ersten und zweiten faserbildenden Materialien synthetische faserbildende Polymere sind.

5. Bikompnnentenfasergeflecht nach Anspruch 4, wobei die faserbildenden Polymere nicht-absorbierbar sind.

6. Bikomponentenfasergeflecht nach Anspruch 1, wobei das faserbildende Polymer Polypropylen (PP), Polyethylen/Tetrafluorethylen-Copolymere (PETFE) oder Polytetrafluorethylen (PTFE) ist.

7. Bikomponenterfasergeflecht nach Anspruch 6, wobei das erste faserbildende Polymer Polypropylen (PP) ist.

8. Bikomponentenfasergeflecht nach Anspruch 7, wobei ein kontinuierliches Filament, das aus dem zweiten faserbildenden Polymer hergestellt ist, eine Festigkeit von mehr als 5,0 g/0,111 tex (denier) aufweist.

9. Bikomponentenfasergeflecht nach Anspruch 8, wobei das zweite faserbildende Polymer Polyethylenterephthalat (PET) oder Polyamid (Nylon) ist.

10. Bikomponentenfasergeflecht nach Anspruch 9, wobei das zweite faserbildende Material Polyethylenterephthalat (PET) ist.

## Revendications

1. Toron de fibres à deux composants comprenant des fils continus et discrets tressés, stérilisés, dans lequel chaque fil est composé de plusieurs filaments, et au moins un filament d'un ou plusieurs fils est une fibre à deux composants, la fibre possédant un fourreau d'une première matière formant fibre englobant sur sa longueur une âme d'une seconde matière formant fibre.

2. Toron de fibres à deux composants de la revendication 1 dans lequel chaque fil est composé de plusieurs fibres à deux composants.

3. Toron de fibres à deux composants de la revendication 2 dans lequel la première et la seconde matières formant fibre sont non-métalliques.

4. Toron de fibres à deux composants de la revendication 3 dans lequel la première et la seconde matières formant fibre sont des polymères synthétiques formant fibre.

5. Toron de fibres à deux composants de la revendication 4 dans lequel les polymères formant fibre sont non-absorbables.

6. Toron de fibres à deux composants de la revendication 1 dans lequel le premier polymère formant fibre est le polypropylène (PP), un copolymère polyéthylène/tétrafluoroéthylène (PETFE) ou le polytétrafluoroéthylène (PTFE).

7. Toron de fibres à deux composants de la revendication 6 dans lequel le premier polymère formant fibre est le polypropylène (PP).

8. Toron de fibres à deux composants de la revendication 7 dans lequel un filament continu préparé à partir du second polymère formant fibre présente une ténacité supérieure à 5,0 grammes/0,111 tex (denier).

9. Toron de fibres à deux composants de la revendication 8 dans lequel le second polymère formant fibre est le téréphtalate de polyéthylène (PET) ou le polyamide (nylon).

10. Toron de fibres à deux composants de la revendication 9 dans lequel la seconde matière formant fibre est le téréphtalate de polyéthylène (PET).
